# EUROPEAN PATENT SPECIFICATION

(11) **EP 0 579 664 B1**
(45) Date of publication and mention of the grant of the patent: **04.02.1998**
(21) Application number: 92908087.7
(22) Date of filing: 13.04.1992
(51) Int. Cl.: C08B 31/18, C07H 7/033, C11D 3/22, D21H 17/28

(54) **A METHOD OF OXIDISING CARBOHYDRATES**
VERFAHREN ZUM OXYDIEREN VON KOHLEHYDRATEN
PROCEDE D'OXYDATION DE GLUCIDES

(30) Priority: 12.04.1991 IT TO910281
(43) Date of publication of application: 26.01.1994
(73) Proprietor: CERESTAR HOLDING B.V., NL-4551 LA Sas van Gent (NL)
(72) Inventor: CONCA, Esterino, I-28100 Novara (IT); BRUSSANI, Gianfranco, I-13051 Biella (IT)
(74) Representative: Wilkinson, Stephen John
(86) International application number: EP9200827
(87) International publication number: WO9218542

(56) References cited:
- EP-A- 0 232 202
- EP-A- 0 247 314
- EP-A- 0 378 127
- US-A- 3 736 224
- US-A- 3 873 614
- US-A- 4 572 798

## Description

The present invention relates to a method of oxidising carbohydrates, particularly starches, dextrin and hydrolysis products thereof.

Oxidised starches are used widely in the paper and textile industries. The product is generally produced by treating starch with hypochlorite in an alkaline aqueous medium. Alternatively, oxidised starches are produced by oxidation with periodate which can cleave the glucoside unit of starch between the C-2 and C-3 atoms which are converted into aldehyde groups. The starch thus produced is used mainly in the production of paper which retains good mechanical strength when wet.

A further potential application of oxidised starch or cellulose, described in German patent application DE-A-24 36 843 is its use as a builder for detergents. The products produced by oxidation with hypochlorite or periodate and subsequently with chlorite contain many carboxylic groups in a chain and thus have good sequestering powers. Their use is limited, however, by the fact that these substances are less biodegradable the higher their degree of oxidation.

US-A-3 736 224 discloses a method whereby lignocellulosic material is treated with oxygen in an alkaline medium in the presence of a copper-containing material suitable copper compounds include complexes of copper with for example 2,2'-bipyridine, 2-carboxypyridine, 2,6-dicarboxypyridine, amino acids. The process is generally conducted at a temperature ranging from 50° to 200°, at atmospheric pressure.

EP-A-0 232 202 discloses a selective oxidation process for polysaccharides by an oxygen containing gas in alkaline medium in the presence of a catalyst based on a noble metal chosen from palladium, platinum, rhodium or osmium fixed on an inert support and doped with one or more metals from the groups IV, V or VI of the periodic table.

The main object of the present invention is to provide an alternative oxidation method which is cheap and advantageous as regards the reagents used in the method. A further object is to provide a method which, with particular reference to the oxidation of starches, gives rise to an oxidation product with improved biodegradability characteristics.

This object is achieved by a method of oxidising carbohydrates, characterised in that the oxidation is effected by means of an oxygen containing gas in an alkaline aqueous medium in the presence of a catalytic quantity of a metal ion selected from the metals of group VIII of the periodic table and silver and a polydentate amine ligand for the metal ion.

Carbohydrates, which constitute the substrate to which the oxidation method of the invention is applied, include starch, hydrolysis products thereof with up to 1 glucoside unit, and simple carbohydrates such as sorbitol.

The term starch essentially means starch which has not been modified chemically and thus includes carbohydrates of natural and vegetable origin in general which are composed essentially of amylose and/or amylopectin. Native starches extracted from various plants such as potatoes, rice, tapioca, maize and cereals may be used. Of these, maize starch is preferred. Hydrolysis products of starch are constituted by mixtures of oligomers with various numbers of glucoside units, including glucose monomer. These hydrolysis products are easily obtainable, for example, by enzymatic hydrolysis, preferably with the use of endoenzymes. Substrates usable for the invention also include polyols with carbohydrate structure, such as sorbitol.

The metal ion used is preferably iron, silver, cobalt or nickel and is introduced into the alkaline aqueous medium by means of a soluble salt, preferably constituted by a chloride or a sulphate.

Typically, the metal ion is used in a molar ratio of between 1 and 0.25% with reference to the number of moles of glucoside units in the substrate.

The ligand for the metal ion is preferably a polydentate amine ligand. Of these the following are contemplated:
- monoamines of the general formula: 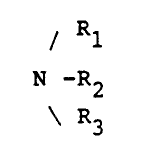 in which one of the radicals R₁, R₂ and R₃ is selected from the group consisting of hydrogen, C₁-C₄ alkyl groups and carboxyalkyl radicals in which the alkyl group has from 1 to 4 carbon atoms, and the rest of the R₁, R₂ or R₃ radicals are the same or different carboxyalkyl radicals in which the alkyl group has from 1 to 4 carbon atoms, and
- polyamines of the general formula: 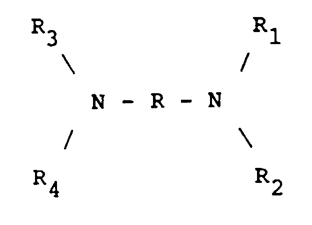 in which R is an alkylene group with from 1 to 4 carbon atoms, preferably ethylene, and

R₁, R₂, R₃, and R₄ are the same or different and are radicals selected independently of each other from the group consisting of hydrogen, C₁-C₄ alkyl groups, aminoalkyl radicals in which the alkyl group has from 1 to 4 carbon atoms and carboxyalkyl radicals in which the alkyl group has from 1 to 4 carbon atoms,
or alternatively, R₁ and R₂ and/or R₃ and R₄ form a heterocyclic ring with the respective nitrogen atom,
or alternatively, R₁, R₂, R₃ and R₄ form heterocyclic groups with the respective nitrogen atoms. Of the preferred amine and polyamine ligands, nitrilotriacetic acid, iminodiacetic acid, ethylenediamine, diethylenetriamine, triethylenetetramine, ethylenediaminetetra-acetic acid (EDTA), ethylenediaminetriacetic acid, phenanthroline and 2,2'-dipyridyl are contemplated in particular.

A combination of EDTA with ferrous sulphate or ferrous chloride is particularly advantageous for the oxidation of starch and hydrolysis products thereof, including glucose.

The oxidation reaction is carried out by bubbling molecular oxygen or air through the alkaline aqueous medium which generally has a pH of from 8 to 14 typically between 8 and 10 at a temperature of from 25 to 90°C and at atmospheric pressure with vigorous stirring.

### Example 1.

40 g of dextrin was dissolved in 500 ml of deionised water. 0.63 g of FeCl₂ and 0.5 g of o-phenanthroline were added. The reaction was carried out at 60°C and at a pH of 9 in an atmosphere of oxygen and good stirring was maintained. A total of 15 ml of 3.4M NaOH was introduced during a reaction period of 8 hours. Upon completion of the reaction the water was evaporated and the product recovered.

### Example 2.

40 g of dextrin was dissolved in 500 ml of deionised water. 0.68 g of FeSO₄.7H₂O and 0.5 g of o-phenanthroline were added. The reaction was carried out at 70°C and at a pH of 9 in an atmosphere of oxygen and good stirring was maintained.

A total of 35 ml of 3.4M NaOH was introduced during a reaction period of 12 hours. Upon completion of the reaction, the water was evaporated and the product recovered.

### Example 3.

40 g of dextrin was dissolved in 500 ml of deionised water. 0.34 g of FeSO₄.7H₂O and 25 g of o-phenanthroline were added. The reaction was carried out at 70°C and at a pH of 9 in an atmosphere of oxygen and good stirring was maintained.

A total of 60 ml of 3.4M NaOH was introduced during a reaction period of 32 hours. Upon completion of the reaction the water was evaporated and the product recovered.

### Example 4.

40 g of dextrin was dissolved in 500 ml of deionised water. 0.34 g of FeSO₄.7H₂O and 0.43 g of the dihydrated disodium salt of EDTA were added. The reaction was carried out at 70°C and at a pH of 9 in an atmosphere of oxygen and good stirring was maintained.

A total of 60 ml of 3.4M NaOH was introduced during a reaction period of 16 hours. Upon completion of the reaction the water was evaporated and the product recovered.

### Example 5.

20 g of soluble starch was dissolved in 500 ml of deionised water. 0.34 g of FeSO₄.7H₂O and 0.25 g of o-phenanthroline were added. The reaction was carried out at 70°C and at a pH of 9 in an atmosphere of oxygen and good stirring was maintained.

A total of 17 ml of 3.4M NaOH was introduced during a reaction period of 12 hours. Upon completion of the reaction the water was evaporated and the product recovered.

### Example 6.

20 g of maize starch was gelled in 500 ml of deionised water. 0.34 g of FeSO₄.7H₂O and 0.46 g of EDTA were added in an atmosphere of oxygen and good stirring was maintained.

A total of 17 ml of 3.4M NaOH was introduced during a reaction period of 17 hours. Upon completion of the reaction the water was evaporated and the product recovered.

The recovered product was subjected to Ft-IR spectroscopy with Perkin Elmer 1760 equipment. The graph below shows the spectra of the oxidation product (2) and of untreated starch (1) in Nujol.

The spectrum (2) has a band at a wavelength of 1597 cm⁻¹ which is characteristic of the salified carboxyl group and is absent from the spectrum (1). The two spectra have substantially corresponding shapes in the region between 1000 and 1100 cm⁻¹ in which there are strong bands characteristic of the structure of starch.

The oxidation product of a starch or a dextrin obtainable by the method which is described above and is the subject of the following claims falls within the scope of the present invention.

The oxidation product of starch may conveniently be used as a binding additive for paper, as a builder for detergents, as a polyelectrolyte thickening agent, in formulations for paints and printing inks, and as a high-molecular-weight coalescent.

Its use as a builder for detergents is particularly advantageous by virtue of its good sequestering properties combined with the biodegradability of the product compared with products oxidised by hypochlorite.

Additionally the product may be used as a co-builder in detergent formulations in association with known builders, such as zeolites, in order to improve the anti-redeposition properties and dispersion capacity of the detergents and achieve an improved soil removal effectiveness.

Hydrolysis products of starch and particularly dextrin oxidised by the method of the invention may also be used in particular as polyelectrolyte thickening agents in formulations for paints and printing inks. These uses constitute a further subject of the invention.

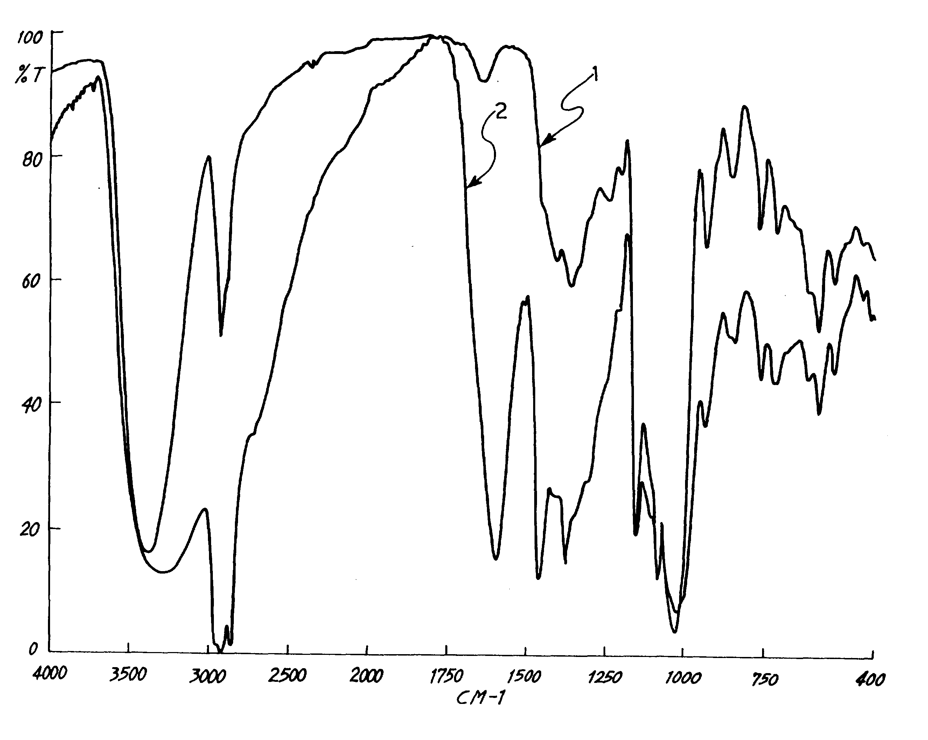

## Claims

1. A method of oxidising carbohydrates, by means of an oxygen containing gas in an alkaline aqueous medium and a metal catalyst characterised in that oxidation is effected in the presence of a catalytic amount of a metal ion selected from the metals of group VIII of the periodic table and silver and of a polydentate amine ligand for the metal ion.

2. A method according to Claim 2, characterised in that the ligand is a monoamine of the general formula: 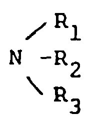 in which one of the radicals R₁, R₂ and R₃ is selected from the group consisting of hydrogen, C₁-C₄ alkyl groups and carboxyalkyl radicals in which the alkyl group has from 1 to 4 carbon atoms and the rest of the R₁, R₂ and R₃ radicals are the same or different carboxyalkyl radicals in which the alkyl group has from 1 to 4 carbon atoms.

3. A method according to Claim 1, characterised in that the ligand is a polyamine of the general formula: 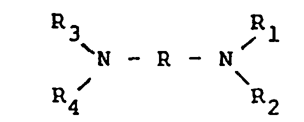 in which R is a C₁-C₄ alkylene group, preferably ethylene, and
R₁, R₂, R₃ and R₄ are the same or different and are selected independently of each other from the group consisting of hydrogen, C₁-C₄ alkyl groups, aminoalkyl radicals in which the alkyl group has from 1 to 4 carbon atoms and carboxyalkyl radicals in which the alkyl group has from 1 to 4 carbon atoms,
or alternatively, R₁ and R₂ and/or R₃ and R₄ form a heterocyclic ring with the respective nitrogen atom,
or alternatively, R₁, R₂, R₃ and R₄ form heterocyclic groups with the respective nitrogen atoms.

4. A method according to Claim 1, characterised in that the amine ligand is selected from the group consisting of nitrilotriacetic acid, iminodiacetic acid, ethylenediamine, diethylenetriamine, triethylenetetramine, ethylenediaminetriacetic acid, ethylenediaminetetra-acetic acid, phenanthroline and 2,2'-dipyridyl.

5. A method according to Claim 4 in which the ligand is ethylenediaminetetra-acetic acid and the metal ion is iron.

6. A method according to Claim 4 in which the metal ion is iron and the ligand is o-phenanthroline.

7. A method according to Claim 1 in which the molar concentration of the metal ion in the reaction medium is from 1 to 0.25% with reference to the number of moles of glucoside in the oxidation substrate.

8. A method according to Claim 1 wherein the oxidation reaction is carried out at a temperature of from 25 to 90°C and at atmospheric pressure with vigorous stirring.

9. A method according to Claim 1 wherein the carbohydrate subjected to oxidation is selected from starch, dextrin, hydrolysis products of starch or dextrin, and sorbitol.

10. An oxidation product of starch, starch hydrolyzate or dextrin obtainable by a method according to any one of Claims 1 to 8.

11. The use of an oxidation product of starch, starch hydrolyzate or dextrin obtainable by a method according to any one of Claims 1 to 8 as a builder or co-builder for detergents.

12. The use of an oxidation product of starch, starch hydrolyzate or dextrin obtainable according to any one of Claims 1 to 8 as a binder for paper, as a polyelectrolyte thickening agent or as a coalescent.

## Patentansprüche

1. Verfahren zum Oxidieren von Kohlenhydraten in einem alkalischen wässerigen Medium mit einem Sauerstoff enthaltenden Gas und einem Metallkatalysator, dadurch gekennzeichnet, daß die Oxidation in Gegenwart einer katalytischen Menge eines Metallions, das aus Metallen der Gruppe VIII des Periodensystems und Silber ausgewählt wird, und eines mehrzähnigen Aminliganden für das Metallion ausgeführt wird.

2. Verfahren nach Anspruch 2, dadurch gekennzeichnet, daß der Ligand ein Monoamin der allgemeinen Formel 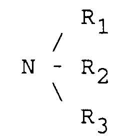 ist, worin einer der Reste R₁, R₂ und R₃ ausgewählt ist aus der Gruppe, bestehend aus Wasserstoff, C₁-C₄-Alkylgruppen und Carboxyalkylresten, in denen die Alkylgruppe 1 bis 4 Kohlenstoffatome aufweist und die übrigen Reste R₁, R₂ und R₃ die gleichen oder verschiedenen Carboxyalkylreste darstellen, in denen die Alkylgruppe 1 bis 4 Kohlenstoffatome aufweist.

3. Verfahren nach Anspruch 1, dadurch gekennzeichnet, daß der Ligand ein Polyamin der allgemeinen Formel 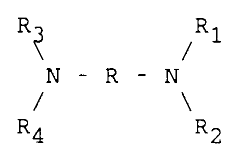
ist, worin R eine C₁-C₄-Alkylengruppe, vorzugsweise Ethylen, darstellt und R₁, R₂, R₃ und R₄ gleich oder verschieden sind und unabhängig voneinander aus der Gruppe, bestehend aus Wasserstoff, C₁-C₄-Alkylgruppen, Aminoalkylresten, in denen die Alkylgruppe 1 bis 4 Kohlenstoffatome aufweist, und Carboxyalkylresten, in denen die Alkylgruppe 1 bis 4 Kohlenstoffatome aufweist, ausgewählt sind
oder alternativ R₁ und R₂ und/oder R₃ und R₄ einen heterocyclischen Ring mit dem betreffenden Stickstoffatom bilden
oder alternativ R₁, R₂, R₃ und R₄ heterocyclische Gruppen mit den betreffenden Stickstoffatomen bilden.

4. Verfahren nach Anspruch 1, dadurch gekennzeichnet, daß der Aminligand aus der Gruppe, bestehend aus Nitrilotriessigsäure, Iminodiessigsäure, Ethylendiamin, Diethylentriamin, Triethylentetramin, Ethylendiamintriessigsäure, Ethylendiamintetraessigsäure, Phenanthrolin und 2,2'-Dipyridyl ausgewählt ist.

5. Verfahren nach Anspruch 4, wobei der Ligand Ethylendiamintetraessigsäure darstellt und das Metallion Eisen darstellt.

6. Verfahren nach Anspruch 4, wobei das Metallion Eisen darstellt und der Ligand o-Phenanthrolin darstellt.

7. Verfahren nach Anspruch 1, wobei die Molkonzentration des Metallions in dem Reaktionsmedium 1 bis 0,25%, bezogen auf die Molzahl an Glucosid in dem Oxidationssubstrat, ist.

8. Verfahren nach Anspruch 1, wobei die Oxidationsreaktion bei einer Temperatur von 25 bis 90°C und bei Atmosphärendruck unter heftigem Rühren ausgeführt wird.

9. Verfahren nach Anspruch 1, wobei das zu oxidierende Kohlenhydrat ausgewählt ist aus Starke, Dextrin, Hydrolyseprodukten von Stärke oder Dextrin und Sorbit.

10. Oxidationsprodukt von Stärke, Stärkehydrolysat oder Dextrin, erhältlich durch ein Verfahren nach einem der Ansprüche 1 bis 8.

11. Verwendung eines Oxidationsprodukts von Stärke, Stärkehydrolysat oder Dextrin, erhältlich durch ein Verfahren nach einem der Ansprüche 1 bis 8, oder als Builder oder Co-builder für Waschmittel.

12. Verwendung eines Oxidationsproduktes von Stärke, Stärkehydrolysat oder Dextrin, erhältlich nach einem der Ansprüche 1 bis 8, als Bindemittel für Papier, als Polyelektrolytverdickungsmittel oder als Koaleszenzmittel.

## Revendications

1. Procédé d'oxydation de glucides, au moyen d'un gaz contenant de l'oxygène dans un milieu aqueux alcalin et d'un catalyseur à base d'un métal, caractérisé en ce que l'oxydation est effectuée en présence d'une quantité catalytique d'un ion métallique choisi parmi les métaux du groupe VIII du Tableau Périodique et l'argent et d'un ligand amine polycoordiné pour l'ion métallique.

2. Procédé suivant la revendication 1, caractérisé en ce que le ligand est une monoamine de formule générale 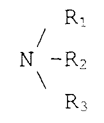 dans laquelle l'un des radicaux R₁, R₂ et R₃ est choisi dans le groupe consistant en l'hydrogène, des groupes alkyle en C₁ à C₄ et des radicaux carboxyalkyle dont le groupe alkyle a 1 à 4 atomes de carbone, et les radicaux R₁, R₂ ou R₃ restants sont des radicaux carboxvalkyle identiques ou différents dans lesquels le groupe alkyle a 1 à 4 atomes de carbone.

3. Procédé suivant la revendication 1, caractérisé en ce que le ligand est une polyamine de formule générale : 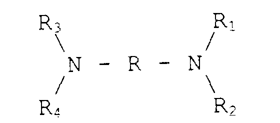 dans laquelle R est un groupe alkylène ayant 1 à 4 atomes de carbone, de préférence un groupe éthylène, et
R₁, R₂, R₃ et R₄ sont identiques ou différents et sont choisis, indépendamment les uns des autres, dans le groupe consistant en l'hydrogène, des groupes alkyle en C₁ à C₄, des radicaux aminoalkyle dont le groupe alkyle a 1 à 4 atomes de carbone, et des radicaux carboxyalkyle dont le groupe alkyle a 1 à 4 atomes de carbone,
ou bien en variante, R₁ et R₂ et/ou R₃ et R₄ forment un noyau hétérocyclique avec l'atome respectif d'azote,
ou en variante, R₁, R₃, R₃ et R₄ forment des groupes hétérocycliques avec les atomes respectifs d'azote.

4. Procédé suivant la revendication 1, caractérisé en ce que le ligand amine est choisi dans le groupe consistant en l'acide nitrilotriacétique, l'acide iminodiacétique, l'éthylènediamine, la diéthylènetriamine, la triéthylènetétramine, l'acide éthylènediaminetriacétique, l'acide éthylènediaminetétra-acétique, la phénanthroline et le 2,2'-dipyridyle.

5. Procédé suivant la revendication 4, dans lequel le ligand est l'acide éthylènediaminetétra-acétique et l'ion métallique est le fer.

6. Procédé suivant la revendication 4, dans lequel l'ion métallique est le fer et le ligand est la o-phénanthroline.

7. Procédé suivant la revendication 1, dans lequel la concentration molaire de l'ion métallique dans le milieu de réaction va de 1 à 0,25 % par rapport au nombre de moles de glucoside dans le substrat à oxyder.

8. Procédé suivant la revendication 1, dans lequel la réaction d'oxydation est conduite à une température de 25 à 90°C et à la pression atmosphérique, sous agitation énergique.

9. Procédé suivant la revendication 1, dans lequel le glucide soumis à l'oxydation est choisi entre l'amidon, la dextrine, des produits d'hydrolyse d'amidon ou de dextrine, et le sorbitol.

10. Produit d'oxydation d'amidon, d'hydrolysat d'amidon ou de dextrine, pouvant être obtenu par un procédé suivant l'une quelconque des revendications 1 à 8.

11. Utilisation d'un produit d'oxydation d'amidon, d'hydrolysat d'amidon ou de dextrine pouvant être obtenu par un procédé suivant l'une quelconque des revendications 1 à 8, comme adjuvant ou co-adjuvant pour détergents.

12. Utilisation d'un produit d'oxydation d'amidon, d'hydrolysat d'amidon ou de dextrine pouvant être obtenu conformément à l'une quelconque des revendications 1 à 8, comme liant pour papier, comme agent épaississant pour polyélectrolyte ou comme agent de coalescence.
